# EUROPEAN PATENT SPECIFICATION

(11) **EP 2 913 022 B1**
(45) Date of publication and mention of the grant of the patent: **01.03.2017**
(21) Application number: 13858014.7
(22) Date of filing: 11.09.2013
(51) Int. Cl.: A61L 27/06, A61L 27/10, A61C 8/00, A61C 13/00

(54) **METHOD FOR MANUFACTURING ONE-PIECE DENTAL IMPLANT**
VERFAHREN ZUR HERSTELLUNG EINES EINTEILIGEN ZAHNIMPLANTATS
PROCÉDÉ DE FABRICATION D'UN IMPLANT DENTAIRE D'UNE SEULE PIÈCE

(30) Priority: 29.11.2012 JP 2012261169
(43) Date of publication of application: 02.09.2015
(73) Proprietor: GC Corporation, Bunkyo-ku Tokyo 113-0033 (JP)
(72) Inventor: NAMETA Yoshinori, Tokyo 160-0008 (JP); TAKAYAMA Masayuki, Tokyo 174-8585 (JP); IKEYA Ryosuke, Tokyo 174-8585 (JP); HIROTA Kazuo, Tokyo 174-8585 (JP)
(74) Representative: Regimbeau
(86) International application number: PCT/JP2013/074470
(87) International publication number: WO 2014/083912

(56) References cited:
- EP-A2- 1 820 467
- WO-A1-2011/125309
- JP-A- H0 970 720
- JP-A- H05 507 222
- JP-A- S63 174 647
- JP-A- S63 240 857
- JP-A- 2000 233 312
- JP-A- 2001 508 686
- JP-A- 2003 339 747
- US-A- 4 738 623
- US-A1- 2005 266 382

## Description

### TECHNICAL FIELD

The present invention relates to a method for manufacturing a one-piece dental implant that is used for dental implant treatment. The one-piece dental implant has an abutment and an implant fixture that are integrated to each other. The implant fixture formed of a titanium or titanium alloy is perforated to have a truncated circular cone-shaped recess part that is tapered from an intraoral side to a jawbone side. The abutment formed of ceramic is firmly adhesively fixed to the recess part of the implant fixture.

### BACKGROUND ART

Conventionally, in a case where teeth are completely or partly missing, treatment using dentures is performed. However, treatment using dental implants is becoming widely used for treating teeth-loss owing to its advantages in function, aesthetics, and maneuverability. In dental implant treatment, an implant fixture is embedded into an embedment hole formed in a part of a jawbone where a tooth is lost, and a dental prosthetic is fixed to its intraoral side by way of an abutment.

As a typical method for fixing an abutment to an implant fixture, there is a method of using a fixing screw that penetrates a through-hole formed in the abutment and fastens a male thread of the fixing screw to a female thread provided to the implant fixture (see, for example, Patent Document 1). However, this method requires the abutment to have a through-hole for the fixing screw. Particularly, in a case where a fixing apparatus of a dental prosthetic, which is the upper structural member, to be fixed to the abutment has a small outer diameter, a part of the abutment for attachment to the fixing apparatus of the dental prosthetic is required to have a small diameter. However, when attempting to ensure a through-hole for a fixing screw, it may be difficult to provide the attachment part of the fixing apparatus of the dental prosthetic of the abutment. Further, even if the attachment part of the fixing apparatus of the dental prosthetic of the abutment can be ensured, the dental prosthetic may be unable to attain a sufficient thickness. As a result, there is a problem of being unable to provide sufficient strength for the dental prosthetic.

Thus, there is proposed a one-piece dental apparatus that has an implant fixture and an abutment integrally formed beforehand without using a fixing screw (see, for example, Patent Document 2). In a case of manufacturing the one-piece dental apparatus by using a typical rod cutting method, the implant fixture and the abutment can only be manufactured by using the same material. Therefore, for example, it is not possible to manufacture a one-piece dental implant in which its implant fixture is made by using titanium or a titanium alloy having satisfactory bioaffinity with bones whereas its abutment is made by using a ceramic such as zirconium oxide having satisfactory aesthetics. Thus, clinical application is extremely limited.

There is also proposed a manufacturing method by combining a ceramic material and a titanium mold body formed with a titanium mold forming compound including titanium or a titanium alloy powder and a binder, and performing a degreasing process or a sintering process thereto and fixing the ceramic material and the titanium mold body to each other (see, for example, Patent Document 3). However, with this method, the manufacturing procedure is complicated compared to the cutting method. In addition, not only is there a possibility of mixing the binder with the ceramic material, but there is also a difficulty of controlling dimension due to shrinking of the titanium mold body.

There is also a problem of peri-implantitis which is a current problem of implant treatment. In a case where there is a fine space at an interface between an implant fixture and an abutment, it has been pointed out that anaerobic bacteria grown in the space may cause bone resorption at the surrounding of an implant and lead to peri-implantitis. Therefore, as a method for attaching an abutment to an implant fixture without creating any space, there is a dental implant in which an engagement hole of an implant fixture and an inserting engagement part of an abutment are both formed having truncated cone shapes with the same taper, so that the implant fixture and the abutment can be engaged by the taper (see, for example, Patent Document 4). With this structure, a space can be prevented from being created at the interface between the implant fixture and the abutment, and peri-implantitis is less likely to occur. However, because the dental implant is expected to have its abutment replaced in a state where the implant fixture remains in the jawbone, there may be a risk of insufficient engagement between the implant fixture and the abutment. Further, because a female screw is to be fastened to the abutment, there is also a problem that the abutment cannot be formed of ceramic such as an zirconium oxide having satisfactory aesthetics but less strength compared to metal.

US 4738623 discloses a dental implant with a root member having a narrowed shoulder at its top, and a head member with a tapered rod or socket for connection to the root.

From EP 1820467 a support post for use with a dental implant is known, which includes a ceramic portion for supporting the prosthesis and a metal insert having a lower portion for engaging the implant; the metal insert and ceramic portion are fixed together by means of a ceramic sealant.

### Related Art Document

### Patent Document

Patent Document 1: Japanese Laid-Open Patent Publication No. 2004-113718
Patent Document 2: Japanese Laid-Open Patent Publication No. 2005-329244
Patent Document 3: Japanese Laid-Open Patent Publication No. 2009-28358
Patent Document 4: Japanese Laid-Open Patent Publication No. 2012-75466

### DISCLOSURE OF THE INVENTION

### PROBLEMS TO BE SOLVED BY THE INVENTION

In view of each of the above-described problems, an embodiment of the present invention is aimed to provide a method for manufacturing a one-piece dental implant that can firmly adhesively fix an implant fixture and an abutment to each other while attaining high fitting accuracy at an interface between the implant fixture and the abutment without having to use fixing members such as a fixing screw or other bonding members. The implant for dental treatment includes the implant fixture being formed of titanium or a titanium alloy having a satisfactory bioaffinity and the abutment being formed of ceramic such as a zirconium oxide having satisfactory aesthetics.

### MEANS OF SOLVING THE PROBLEMS

As a result of diligent research for solving the above-described problems, the inventors of the present invention have conceived the present invention in which a one-piece dental implant having a ceramic abutment and a titanium or titanium alloy implant fixture firmly adhesively fixed to each other while attaining high fitting accuracy at the interface between the abutment and the implant fixture by cooling the implant fixture to room temperature after a projecting part of the abutment at room temperature is press-fitted into a recess part of the implant fixture being in a state where the implant fixture is heated to 100-300 °C. The implant fixture formed of titanium or a titanium alloy is perforated to have a truncated circular cone-shaped recess part. The recess part has a taper that is tapered from an intraoral side to a jawbone side. The abutment formed of ceramic has a prosthetic fixing part provided on an opposite side of the jawbone side and a truncated circular cone-shaped projecting part provided on the jawbone side. The projecting part has the same taper as the recess part of the implant fixture. Further, it is conceived that a high fitting accuracy can be attained at the interface between the abutment and the implant fixture when both the truncated circular cone-shaped recess part of the implant fixture and the truncated circular cone-shaped projecting part of the abutment have a taper angle of 0.5-6 ° in a case of using this method to manufacture the one-piece dental implant having the abutment and the implant fixture integrated to each other.

That is, an embodiment of the present invention provides a one-piece implant having an abutment and an implant fixture integrated to each other and is manufactured by cooling the implant fixture to room temperature after a projecting part of the abutment at room temperature is press-fitted into a recess part of the implant fixture being in a state where the implant fixture is heated to 100-300 °C. The implant fixture formed of titanium or a titanium alloy is perforated to have a truncated circular cone-shaped recess part. The recess part has a taper that is tapered from an intraoral side to a jawbone side. The abutment formed of ceramic has a prosthetic fixing part provided on an opposite side of the jawbone side and a truncated circular cone-shaped projecting part provided on the jawbone side. The projecting part has the same taper as the recess part of the implant fixture. Further, the method for manufacturing the one-piece dental implant is preferable when both the truncated circular cone-shaped recess part of the implant fixture and the truncated circular cone-shaped projecting part of the abutment have a taper angle of 0.5-6 ° .

### EFFECTS OF THE INVENTION

An embodiment of the present invention can provide a method for manufacturing a one-piece dental implant that can adhesively fix an implant fixture and an abutment to each other while attaining high fitting accuracy at an interface between the implant fixture and the abutment without having to use fixing members such as a fixing screw or other bonding members. Therefore, in a case where a fixing apparatus of a dental prosthetic, which is the upper structural member, to be fixed to the abutment has a small outer diameter, it is possible to use a ceramic as the abutment. The implant for dental treatment includes the implant fixture being formed of titanium or a titanium alloy having a satisfactory bioaffinity and the abutment being formed of ceramic such as a zirconium oxide having satisfactory aesthetics.

### BRIEF DESCRIPTION OF DRAWINGS

Fig. 1 is a cross-sectional explanatory diagram illustrating an implant fixture to be used in a method for manufacturing a one-piece dental implant according to an embodiment of the present invention;
Fig. 2 is a cross-sectional explanatory diagram illustrating an abutment to be used in a method for manufacturing a one-piece dental implant according to an embodiment of the present invention; and

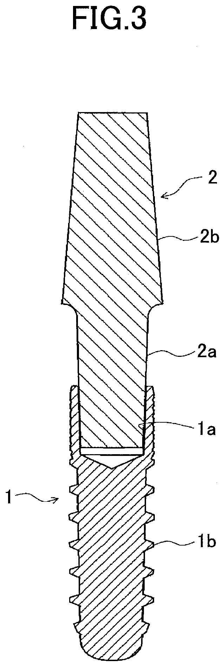

Fig. 3 is a cross-sectional explanatory diagram illustrating a state where the implant fixture of Fig. 1 and the abutment of Fig. 2 are manufactured by using a method for manufacturing a one-piece dental implant according to an embodiment of the present invention.

### EMBODIMENTS FOR CARRYING OUT THE INVENTION

Next, a method for manufacturing a one-piece dental implant according to an embodiment of the present invention is described with reference to the accompanying drawings.

In the drawings, reference numeral 1 is an implant fixture that is embedded into a cavity formed in a tooth-missing part of a jawbone and has a dental prosthetic fixed thereto interposed by an abutment on its intraoral side. The implant fixture 1 is formed of pure titanium or titanium alloy having satisfactory bioaffinity. A pure titanium of JIS grade 2 or 4 is widely used as the pure titanium. As the type of titanium alloy, TiAl4V alloy, Ti6Al7Nb alloy or the like is used. The implant fixture 1 is perforated to have a truncated circular cone-shaped recess part 1a including a taper that becomes narrower from an intraoral side to a jawbone side. A male thread 1b is often provided at an outer periphery of the implant fixture 1 on its jawbone side.

Reference numeral 2 is an abutment formed of ceramic such as zirconium oxide having satisfactory aesthetics and high strength. The abutment 2 has a prosthetic fixing part 2b provided on a side opposite of the jawbone side and a truncated circular cone-shaped projecting part 2a provided on the jawbone side. The projecting part 2a has the same taper as the truncated circular cone-shaped recess part 1a of the implant fixture 1. Although yttria partially stabilized zirconia is widely known to be commonly used as zirconia, a ceria partially stabilized zirconia or a complex oxide with alumina or the like may also be used. It is preferable that at least a part of a transverse section of the prosthetic fixing part 2b of the abutment 2 is a non-circular shape. This is because the part having the non-circular-shaped transverse section serves as a part for transmitting force from an implant embedding driver or the like. Further, before the implant that is manufactured by using the method for manufacturing a one-piece dental implant according to an embodiment of the present invention is embedded into a cavity formed in a tooth-missing part of a jawbone, the abutment 2 and the implant fixture 1 are integrated to each other. Thus, it is also preferable because a direction of a dental prosthetic fixed to the abutment 2 can be regulated in a state where the implant is embedded into the cavity formed in the tooth-missing part of the jawbone. The truncated circular cone-shaped projecting part 2a of the abutment 2 includes a part that is fixed to the implant fixture 1 by being pressingly inserted into the recess part 1a of the implant fixture 1 and a part that contacts a gingiva between the prosthetic fixing part 2b and an intraoral side of the recess part 1a of the implant fixture 1. Thus, the truncated circular cone-shaped projecting part 2a is to be formed of ceramic in order to ensure that is not deteriorated aesthetics, for example, in a case of being exposed the part in in contact with the gingiva due to gingiva recession.

With the method for manufacturing a one-piece dental implant according to an embodiment of the present invention, the implant fixture 1 is cooled to room temperature after the projecting part 2a of the abutment 2 is press-fitted into the recess part 1a of the implant fixture 1 in a state where the implant fixture 1 is heated to 100-300 °C. Thereby, a one-piece dental implant can be manufactured to have the abutment 2 and the implant fixture 1 firmly adhesively fixed to each other to form an integrated body. The implant fixture 1 is to be heated to a temperature of 100-300 °C because the expanding effect of the recess part 1a of the implant fixture 1 is weak if the temperature is less than 100 °C whereas operating danger increases if the temperature exceeds 300 °C. Thus, the temperature is preferably 120-200 °C.

In the above-described operation, because both the projecting part 2a of the abutment 2 and the recess part 1a of the implant fixture 1 are formed to have truncated circular cone-shapes having the same taper, the projecting part 2a of the abutment 2 can firmly engage the recess part 1a of the implant fixture 1 over a wide area by press-fitting the projecting part 2a of the abutment 2 having room temperature into the recess part 1a of the implant fixture 1 that is widened by the heating. Thereby, even in a case where the abutment 2 is formed of ceramic having less strength compared to metal, a one-piece dental implant can be manufactured to have the abutment 2 and the implant fixture 1 firmly adhesively fixed to each other to form an integrated body.

A taper angle (defined as an angle formed by a taper surface and a center axis of the abutment 2 in a vertical section including the center axis of the abutment 2) of the projecting part 2a of the abutment 2 and the recess part 1a of the implant fixture 1 having the same taper is 0.5-6° , and more preferably 1-3° . It is undesirable for the taper angle to be less than 0.5° because inserting the projecting part 2a of the abutment 2 into the recess part 1a of the implant fixture 1 would be difficult due to no significant difference between the diameter of a jawbone-side tip of the recess part 2a of the abutment 2 and the diameter of the recess part 1a of the implant fixture 1 on its intraoral side. It is undesirable for the taper angle to exceed 6° because the projecting part 2a of the abutment 2 may be damaged due to a significant difference between the contractive force at the intraoral side of the recess part 1 and the contractive force at the jawbone side of the recess part 1 when the implant fixture 1 is cooled to room temperature after heating the implant fixture 1 to 100-300 °C and press-fitting the projecting part 2a of the abutment 2 into the recess part 1a of the implant fixture 1.

The method for press-fitting the projecting part 2a into the recess part 1a is not limited in particular. For example, the press-fitting method may be performed by erecting the abutment 2 on a simple mold in a state where the projecting part 2a is oriented upward, engaging the recess part 1a of a heated implant fixture 1 from above the abutment 2, and using a drilling machine or the like. In this process, the abutment 2 and the implant fixture 1 can engage deeper compared to a case without heating because the recess part 1a is in a slightly widened state owing to the implant fixture 1 being expanded by heating. Thus, an effect of more firmly integrating the abutment 2 and the implant fixture 1 can be attained. After the press-fitting, the implant fixture 1 returns to room temperature by allowing the implant fixture 1 to cool naturally. Because a typical implant fixture has a diameter of 2-10 mm, it is preferable to perform the press-fitting method by inserting the projecting part 2a of the abutment 2 into the recess part 1a of the implant fixture 1 and applying a force of 40-100 kgf.

### Working example

Next, the present invention is described in detail with reference to a working example. However, the present invention is not limited to the working example.

### <Working example>

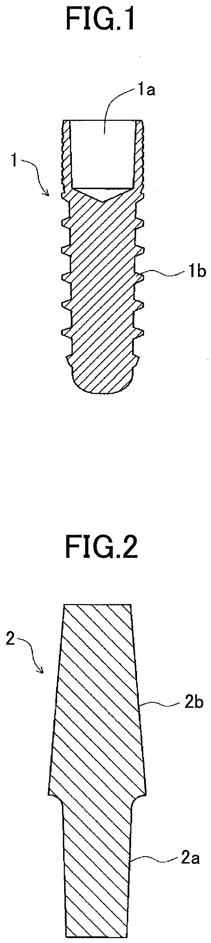

Ti6Al4V was selected as the titanium alloy, and a rod member thereof was fabricated into the implant fixture 1. The implant fixture 1 was cut to have a diameter of 3 mm and a length of 10mm. The truncated circular cone-shaped recess part 1a for engaging the abutment 2 was formed in the implant fixture 1 on the intraoral side of the implant fixture 1. The recess part 1a was formed with a taper angle of 2° from a part where the diameter is 2.4 mm to a depth of 3mm. See Fig. 1. Meanwhile, the abutment 2 having the projecting part 2a on a jawbone side and a truncated quadrangular cone-shaped prosthetic fixing part 2b on an intraoral side was fabricated by processing a powder of yttria partially stabilized zirconia inside a mold and applying isotropic pressure of 1 t to form a molded body, and sintering the molded body at 1300°C for 3 hours. The projecting part 2a was formed to have a length of 5 mm with a taper angle of 2° , and the prosthetic fixing part 2b was formed to have a length of 6 mm. See Fig. 2. <Process of press-fitting integration> The abutment 2 is erected and fixed on a simple mode in a state where the projecting part 2a is oriented upward. Then, the recess part 1a of the implant fixture 1 that has been heated to 150°C in a drying machine is engaged to the projecting part 2a from above the abutment 2 and applied with pressure of 60 kgf from a drilling machine. In the press-fitted state, the implant fixture 1 is cooled to room temperature. Thereby, a one-piece dental implant can be manufactured to have the abutment 2 and the implant fixture 1 firmly adhesively fixed to each other to form an integrated body. According to an observation with a microscope, no space was identified at the interface between the abutment 2 and the implant fixture 1 of the manufactured one-piece dental implant, and an extremely satisfactory fitting accuracy was confirmed. Further, no space was observed at the interface between the abutment 2 and the implant fixture 1 even in a case where a moment of 25 Ncm is applied to an intraoral-side end part of the abutment 2 in a transverse direction relative to its axis.

### <Comparative example>

A one-piece dental implant was manufactured by fabricating the implant fixture 1 and the abutment 2 in the same manner as the working example and engaging the projecting part 2a of the abutment 2a to the recess part 1a of the implant fixture 1 by applying pressure of 60 kgf to press-fit the projecting part 2a of the abutment 2 into the recess part 1a of the implant fixture 1 having room temperature. According to an observation with a microscope after the manufacturing, no space was identified at the interface between the abutment 2 and the implant fixture 1, and an extremely satisfactory fitting accuracy was confirmed. However, when a moment of 1 Ncm was further applied to an intraoral side end part of the abutment 2 in a transverse direction relative to its axis, a space greater than or equal to 5 *µ* m was observed at the interface between the abutment 2 and the implant fixture 1.

Hence, with the working example, no space was observed at the interface between the abutment and the implant fixture even in a case where a moment of 25 Ncm was applied to the intraoral side end part of the abutment, and a high fitting accuracy was confirmed. On the other hand, with the comparative example, a space greater than or equal to 5 *µ* m was confirmed in a case where a moment of 1 Ncm was applied. Accordingly, with the method for manufacturing a one-piece dental implant according to an embodiment of the present invention, a one-piece dental implant can be manufactured to have high fitting accuracy at the interface between the abutment and the implant fixture and firmly adhesively integrate the abutment and implant fixture.

### DESCRIPTION OF THE REFERENCE NUMERALS

- 1: implant fixture
- 1a: truncated circular cone-shaped recess part
- 1b: male thread
- 2: abutment
- 2a: truncated circular cone-shaped projecting part
- 2b: prosthetic fixing part

## Claims

1. A method for manufacturing a one-piece implant having an abutment (2) and an implant fixture (1) integrated to each other, the method comprising:
cooling the implant fixture (1) to room temperature after a projecting part (2a) of the abutment (2) at room temperature is press-fitted into a recess part (1a) of the implant fixture (1) being in a state where the implant fixture (1) is heated to 100-300 °C,
wherein the implant fixture (1) formed of titanium or a titanium alloy is perforated to have a truncated circular cone-shaped recess part (1a), the recess part (1a) having a taper that is tapered from an intraoral side to a jawbone side,
wherein the abutment (2) formed of ceramic has a prosthetic fixing part (2b) provided on an opposite side of the jawbone side and a truncated circular cone-shaped projecting part (2a) provided on the jawbone side, the projecting part (2a) having the same taper as the recess part (1a) of the implant fixture (1).

2. The method for manufacturing a one-piece implant as claimed in claim 1,
wherein both the truncated circular cone-shaped recess part (1a) of the implant fixture (1) and the truncated circular cone-shaped projecting part (2a) of the abutment (2) have a taper angle of 0.5-6 ° .

## Patentansprüche

1. Verfahren zur Herstellung eines einteiligen Implantats, das einen Anschlag (2) und eine damit integrierte Implantathalterung (1) aufweist, das Verfahren umfassend:
Abkühlen der Implantathalterung (1) auf Raumtemperatur, nach dem Einpassen eines vorstehenden Teils (2a) des Anschlags (2) bei Raumtemperatur mit Presssitz in einen Aussparungsteil (1a) der Implantathalterung (1), der sich in einem Zustand befindet, in dem die Implantathalterung (1) auf 100-300°C erhitzt ist,
wobei die aus Titan oder einer Titanlegierung geformte Implantathalterung (1) perforiert ist, um einen kreiskegelstumpfförmigen Aussparungsteil (1a) aufzuweisen, wobei der Aussparungsteil (1a) einen sich von einer intraoralen Seite zu einer Kieferknochenseite verjüngenden Kegel aufweist,
wobei der Anschlag (2), aus Keramik geformt, einen prothetischen Befestigungsteil (2b) aufweist, der auf einer gegenüberliegenden Seite der Kieferknochenseite bereitgestellt ist, und einen auf der Kieferknochenseite kreiskegelstumpfförmigen vorstehenden Teil (2a) bereitgestellt ist, wobei der vorstehenden Teil (2a) der gleichen Verjüngung wie der Aussparungsteil (1a) der Implantathalterung (1) aufweist.

2. Verfahren zur Herstellung eines einteiligen Implantats nach Anspruch 1,
wobei sowohl der kreiskegelstumpfförmigen Aussparungsteil (1a) der Implantathalterung (1) als auch der kreiskegelstumpfförmigen vorstehenden Teil (2a) des Anschlags (2) einen Kegelwinkel von 0,5-6° aufweisen.

## Revendications

1. Procédé pour fabriquer un implant d'un seul tenant ayant une butée (2) et un dispositif de fixation d'implant (1) intégrés l'un par rapport à l'autre, le procédé comprenant :
le refroidissement du dispositif de fixation d'implant (1) à température ambiante après qu'une partie en saillie (2a) de la butée (2) à température ambiante a été montée à la presse dans une partie d'évidement (1a) du dispositif de fixation d'implant (1) qui est dans un état dans lequel le dispositif de fixation d'implant (1) est chauffé à 100 - 300 °C,
dans lequel le dispositif de fixation d'implant (1) formé à partir de titane ou d'un alliage de titane est perforé pour avoir une partie d'évidement de forme tronconique circulaire (1a), la partie d'évidement (1a) ayant un cône qui est progressivement rétréci d'un côté intrabuccal à un côté de mâchoire,
dans lequel la butée (2) formée à partir de céramique a une partie de fixation prothétique (2b) prévue sur un côté opposé d'un côté de mâchoire et une partie en saillie de forme tronconique circulaire (2a) prévue du côté de la mâchoire, la partie en saillie (2a) ayant le même cône que la partie d'évidement (1a) du dispositif de fixation d'implant (1).

2. Procédé pour fabriquer un implant d'un seul tenant selon la revendication 1,
dans lequel à la fois la partie d'évidement de forme tronconique circulaire (1a) du dispositif de fixation d'implant (1) et la partie en saillie de forme tronconique circulaire (2a) de la butée (2) ont un angle de conicité de 0,5 - 6°.
